# EUROPEAN PATENT APPLICATION

(11) **EP 4 176 890 A1**
(43) Date of publication of application: **10.05.2023**
(21) Application number: 21306546.9
(22) Date of filing: 03.11.2021
(51) Int. Cl.: A61K 35/747, A61K 35/745, A61P 25/24, A61P 25/22, A61K 38/16

(54) **OXYTOCIN-MIMETIC COMPOSITIONS AND USES THEREOF**

(71) Applicant: INSERM (Institut National de la Santé et de la Recherche Médicale), 75654 Paris Cedex 13 (FR); Université de Rouen, 76130 Mont-Saint-Aignan (FR); Targedys, 91160 Longjumeau (FR)
(72) Inventor: FETISSOV, Seguei, 76380 Montigny (FR); NICOL, Marion, 76230 Bois-Guillaume (FR); PICOLO, Clémentine, 76000 Rouen (FR); LAMBERT, Grégory, 76000 Rouen (FR)
(74) Representative: Icosa

(57) **Abstract**

The present invention relates to compositions comprising *Lactobacillus salivarius* strain and least one second *Lactobacillus* strain, and optionally at least one *Bifidobacterium* strain. The invention further relates to the nutraceutical and therapeutical uses of such compositions in mood management.

## Description

### FIELD OF INVENTION

The present invention relates to a composition comprising at least one Lactobacillus and optionally at least one Bifidobacterium for use in oxytocin-related disorders. The invention further relates to the use of the composition in the mood management.

### BACKGROUND OF INVENTION

According to the World Health Organization (2020), depression affects more than 264 million people worldwide. Many of those also suffer from anxiety. This does not only have a negative impact in the health of those suffering from them, but also on their life quality. Current research states that both disorders are triggered by the interaction of psychological, environmental, genetic, and biological factors. There are many therapeutic options to treat these disorders. However, these options often take a long time to work, cause mood swings, alterations in sleeping patterns, dependence and addiction, and health affections in other parts of the body.

Thus, there is a need to supply compositions for their use in treating neurocognitive dysfunctions such as depression and/or anxiety.

Recent scientific advances have shown a link between the gut microbiota and the host central nervous system, and the communication between them occurs via a bidirectional pathway termed the "microbiota-gut-brain axis.". For instance, the international application WO2015/082633 discloses the "microbiota-gut-brain axis" cross-link between gut bacteria and the host nervous system via bacterial protein mimetics of neuropeptides. Indeed, caseinolytic protease B (ClpB) of Enterobacteriaceae has been identified as a conformational mimetic of alpha-melanocyte-stimulating hormone (alpha-MSH), a neuropeptide regulating feeding behavior and emotion in the context of eating disorders. Nevertheless, the general aspects of the links between the gut microbiota and depression have not been systematically investigated.

Oxytocin (OT) is a neuropeptide involved in a variety of physiological functions including key roles in stress, emotion, mood, social and feeding behavior and displays anti-stress, anxiolytic, pro-social, analgesic and anti-depressive effects. The inventors, surprisingly found bacterial strains that act on the microbiota-gut-brain axis and thus neurocognitive functions via the oxytocin pathway and propose novel compositions for use in the treatment of neuropsychiatric dysfunctions.

The gut microbiome in the modern environment has markedly changed in response to natural and technological factors. These changes may impact on a broad range of host physiology including mood namely in view of the every-day stress that humans are subjected to.

The present invention further addresses this problem by supplying nutraceutical and pharmaceutical uses of the compositions detailed hereinbelow to ensure the well-being of a subject.

### SUMMARY

The invention relates to a composition for use in the treatment of depression; said composition comprising *Lactobacillus salivarius* strain and at least one second *Lactobacillus* strain, and optionally at least one *Bifidobacterium* strain.

In one embodiment, the at least one second *Lactobacillus* strain is selected from *Lactobacillus gasseri, Lactobacillus camelliae, Lactobacillus plantarum,* and/or *Lactobacillus reuteri.*

In one embodiment, the at least one *Bifidobacterium* strain is selected from *Bifidobacterium ruminantium, Bifidobacterium dentium, Bifidobacterium pseudolongum* subsp. *pseudologum, Bifidobacterium pseudolongum* subsp. *globosum* and/or *Bifidobacterium adolescentis.*

According to a first embodiment, the composition comprises *Lactobacillus salivarius* and *Lactobacillus gasseri.*

According to a second embodiment, the composition comprises *Lactobacillus salivarius, Lactobacillus camelliae* and *Bifidobacterium ruminantium.*

The invention further relates to the use of a composition for improving the mood and/or for relieving the every-day stress of a healthy subject, said composition comprising *Lactobacillus salivarius* strain and least one second *Lactobacillus* strain, and optionally at least one *Bifidobacterium* strain. According to a first embodiment of such use, the composition comprises *Lactobacillus salivarius* and *Lactobacillus gasseri.* According to a second embodiment of such use, the composition comprises *Lactobacillus salivarius, Lactobacillus camelliae* and *Bifidobacterium ruminantium.*

In one embodiment, the composition further comprises at least one nutraceutically acceptable excipient.

The invention also relates to a composition comprising *Lactobacillus salivarius* strain, at least one second *Lactobacillus* strain and optionally at least one *Bifidobacterium* strain.

In one embodiment, the at least one second *Lactobacillus* strain is *Lactobacillus gasseri.*

In one embodiment, the at least one second *Lactobacillus* strain is *Lactobacillus camelliae* and the composition comprises at least one *Bifidobacterium* strain selected from *Bifidobacterium ruminantium.*

In one embodiment, the composition further comprises at least one plant or plant extract, preferably selected from the group consisting of *Rhodiola rosea, Withania somnifera, Melissa officinalis, Crocus sativus* and *Hypericum perforatum* and/or at least one magnesium supplement preferably selected from magnesium bound to an ammo acid or a magnesium salt selected from the group consisting of magnesium carbonate, magnesium oxide, magnesium acetate, magnesium ascorbate, magnesium citrate, magnesium gluconate, magnesium lactate, magnesium malate, magnesium pyrrolidone carboxylate, magnesium taurate, and magnesium threonate.

The composition according to the invention may be formulated in an oral dosage form selected from enterically-coated tablets and enterically-coated capsules, wherein the enteric-coating is a mixture comprising hydroxypropyl methyl cellulose and gellan gum.

The composition according to the invention may be also be formulated in a moisture-tight blister or container.

Lastly, the invention relates to a composition comprising at least one bioactive peptide, wherein the at least one bioactive peptide is selected from the group consisting of peptides presenting from at least 80%, from at least 85%, from at least 90 from at least 95%, or 100% identity with any one the peptides of SEQ ID 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42, SEQ ID NO 43, and/or SEQ ID NO 44, for use in the prevention and/or treatment of depression.

### DEFINITIONS

In the present invention, the following terms have the following meanings:
"About" preceding a figure means plus or less 10% of the value of said figure, preferably plus or less 5% of the value of said figure, in particular plus or less 1% of the value of said figure.

**"Excipients"** refers to any inactive ingredient which is required for the formulation of an active agent in a suitable dosage form. Excipients are materials suitable for administration and include any such material known in the art which is non-toxic and which does not interact with any components of the composition in a deleterious manner. In one embodiment, "Excipients" refers to any and all solvents, diluents carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, or any combination thereof.

**"Food"** refers to liquid (i.e. drink), solid or semi-solid dietetic compositions, especially total food compositions (food-replacement), which do not require additional nutrient intake or food supplement compositions. Food supplement compositions do not completely replace nutrient intake by other means. Food and food supplement compositions are for example fermented dairy products or dairy-based products, which are preferably administered or ingested orally one or more times daily. Fermented dairy products can be made directly using the bacteria according to the invention in the production process, e.g. by addition to the food base, using methods known per se. In such methods, the strain(s) of the invention may be used in addition to the micro-organism usually used, and/or may replace one or more or part of the micro-organism usually used. For example, in the preparation of fermented dairy products such as yoghurt or yoghurt-based drinks, a combination of bacteria of the invention may be added to or used as part of a starter culture or may be suitably added during such a fermentation. Optionally, the bacteria may be inactivated or killed later in the production process. Fermented dairy products include milk-based products, such as (but not limited to) deserts, yoghurt, yoghurt drinks, quark, kefir, fermented milk-based drinks, buttermilk, cheeses, dressings, low fat spreads, fresh cheese, soy-based drinks, ice cream, etc. Alternatively, food and/or food supplement compositions may be non-dairy or dairy non fermented products (e.g. strains or cell-free medium in non-fermented milk or in another food medium). In some embodiments, the strains used in the present invention are encapsulated and dispersed in a food (e.g. in milk) or non-food medium. Non-fermented dairy products may include ice cream, nutritional bars and dressings, and the like. Non-dairy products may include powdered beverages and nutritional bars, and the like. The products may be made using known methods, such as adding an effective amount of the strain(s) and/or cell-free culture medium to a food base, such as skimmed milk or milk or a milk-based composition and fermentation as known. Other food bases to which the (compositions comprising the) bacterial cells and/or cell-free culture medium may be added are meat, meat replacers or plant bases.

**"Food ingredient"** or **"feed ingredient"** refers to a formulation which is or can be added to functional foods or foodstuffs as a nutritional supplement.

**"Nutritional food"** or **"nutraceutical"** or **"functional"** food refers to a foodstuff which contains ingredients having beneficial effects for health or capable of improving physiological functions.

**"Food supplement"** refers to a foodstuff having the purpose of completing normal food diet. A food supplement is a concentrated source of nutrients or other substances having a nutritional or physiological effect, when they are taken alone or as a combination in small amounts.

**"Pharmaceutical composition"** refers to a composition comprising an active principle in association with a pharmaceutically acceptable vehicle or excipient. A pharmaceutical composition is for therapeutic use, and relates to health. Especially, a pharmaceutical composition may be indicated for treating a disease or disorder.

By **"pharmaceutically acceptable"** is meant that the ingredients of a pharmaceutical composition are compatible with each other and not deleterious to the subject to which it is administered.

**"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA. The terms **"pharmaceutically acceptable excipient", "pharmaceutically acceptable carrier"** or **"pharmaceutical vehicle"** refer to an inert medium or carrier used as a solvent or diluent in which the pharmaceutically active ingredient is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as the FDA or EMA. For the purposes of the invention, **"pharmaceutically acceptable excipient"** includes all pharmaceutically acceptable excipients as well as all pharmaceutically acceptable carriers, diluents, and/or adjuvants.

**"Pharmaceutically effective amount"** refers to the amount of a pharmaceutical composition necessary and sufficient for slowing down or stopping the progression, aggravation, or deterioration of one or more symptoms of a disease, disorder or condition; or alleviating the symptoms of a disease, disorder or condition; or curing the disease, disorder or condition.

**"Nutraceutical composition"** refers to a composition comprising an edible ingredient allegedly providing a physiological benefit. A nutraceutical composition is for non-therapeutic use, and relates to comfort. The term "comfort" refers to the feeling of ease or well-being. Especially, a nutraceutical composition may be used for promoting, maintaining and/or improving comfort or for alleviating and/or preventing a discomfort. A nutraceutical composition may be in the form of a nutritional product, such as, for example, a functional food or a food or dietary supplement.

**"Nutraceutically effective amount"** refers to the amount of a nutraceutical composition, food or dietary supplement or functional food necessary and sufficient for providing a physiological benefit or alleviating a discomfort.

The term **"administration",** or a variant thereof (e.g., **"administering"),** means providing the active agent, herein the strain combination, alone or as part of a pharmaceutically acceptable composition, to the patient in whom/which the condition, symptom, or disease is to be treated.

The term **"subject"** refers to a mammal, preferably a human. According to the present invention, a subject is a mammal, preferably a human. In one embodiment, the subject is a "patient", i.e., a mammal, preferably a human, who/which is awaiting the receipt of, or is receiving medical care or was/is/will be the object of a medical procedure or is monitored for the development of a condition, symptom, disorder or disease.

The term **"human"** refers to a subject of both genders and at any stage of development (*i.e.,* neonate, infant, juvenile, adolescent, adult).

The term **"therapeutically effective amount"** (or more simply an **"effective amount")** refers to the amount of active agent, herein the strain combination, alone or as part of a pharmaceutical composition, that is aimed at, without causing significant negative or adverse side effects to the subject in need of treatment, preventing, reducing, alleviating or slowing down (lessening) one or more of the symptoms of a condition, symptom, disorder or disease.

The terms **"treat", "treating"** or **"treatment",** as used herein, refer to a therapeutic treatment, to a prophylactic (or preventive) treatment, or to both a therapeutic treatment and a prophylactic (or preventive) treatment, wherein the object is to prevent, reduce, alleviate, and/or slow down (lessen) one or more of the symptoms of a condition, symptom, disorder or disease, in a subject in need thereof.

### DETAILED DESCRIPTION

### Strains' compositions

The present invention relates to compositions comprising bacteria which produce oxytocin-like immunoreactive proteins.

In one embodiment, the composition comprises at least two strains that express at least two, preferably at least three, even more preferably at least four, of the proteins selected from the group of SEQ ID NO: 1 to SEQ ID NO:26. In one embodiment, the composition comprises at least two strains that express at least two, preferably at least three, even more preferably at least four, of the proteins that present at least one of the sequences selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:41 and SEQ ID NO:44. In one embodiment, the at least two strains are selected from the group consisting of *Bifidobacterium ruminantium, Bifidobacterium pseudolongum* subsp. *globosum, Bifidobacterium pseudolongum* subsp. *pseudolongum, Bifidobacterium dentium, Lactobacillus reuteri, Lactobacillus plantarum, Lactobacillus camelliae, Lactobacillus gasseri,* and/or *Lactobacillus salivarius.* In one embodiment, the at least two strains are selected from the group consisting of *Bifidobacterium ruminantium, Lactobacillus camelliae, Lactobacillus gasseri,* and/or *Lactobacillus salivarius.*

In an embodiment, the invention relates to a composition comprising at least two *Lactobacillus* strains, said strains expressing at least one protein presenting an oxytocin-like activity. "Mimetic" refers to a protein that imitates another protein. This imitation is possible since the protein shares certain characteristics with the protein it mimics.

In one embodiment, oxytocin-like activity refers to immune conformational mimetism wherein the at least one protein cross-reacts with anti-oxytocin antibodies. In one embodiment, oxytocin-like activity refers to the oxytocin receptor agonism wherein the at least one protein, typically a fraction thereof, activates the oxytocin receptor. In one embodiment, oxytocin-like activity refers to immune conformational mimetism and/or oxytocin receptor agonism. A "conformational mimetic" refers to a protein, that shares at least in part the same conformation as another protein.

In one embodiment, the composition comprises at least one *Lactobacillus salivarius* strain and at least one second *Lactobacillus* strain and optionally at least one *Bifidobacterium* strain. In one embodiment, the composition comprises *Lactobacillus salivarius* and at least one second *Lactobacillus* strain, and optionally at least one *Bifidobacterium* strain.

*Lactobacillus salivarius* is a Gram-positive non-sporulating bacterial species. It is a homofermentative organism that produces only a by-product of metabolism, lactic acid, and is naturally found in the human oral cavity, intestines and vaginal mucosa. It is considered non-pathogenic, and is sometimes used to produce lactic acid in fermented foods, probiotics, to help prevent infections with other microorganisms. In humans, *L. salivarius* has been used to prevent and treat a variety of diseases such as asthma, cancer, atopic dermatitis and halitosis, and to a limited extent, to prevent or treat infections (Chaves et al., J. Appl. Microbiol. 2017; 123(1), pp. 18-28). According to the scientific review by Chaves et al., it seems that the use, in particular the oral use such as the oral probiotic use, of *L. salivarius* does not pose a health risk to animals or humans in the doses currently used for a variety of applications.

The inventors found out that ***L. salivarius* expresses the following proteins** that surprisingly immune cross-react with anti-oxytocin antibodies and thus present an oxytocin-like effect:
- Elongation factor Tu having an amino acid sequence of SEQ ID NO: 1,
- Glucose-6-phosphate isomerase having an amino acid sequence of SEQ ID NO:2, and
- Phosphoglycerate kinase having an amino acid sequence of SEQ ID NO:3.

In one specific embodiment, *L. salivarius* is the *L. salivarius* DSM 16530 strain.

According to a first embodiment, the at least one second Lactobacillus strain is selected from the group consisting of *Lactobacillus gasseri, Lactobacillus camelliae, Lactobacillus plantarum,* and/or *Lactobacillus reuteri.*

*Lactobacillus gasseri* is a lactic acid bacterium from the Lactobacillaceae family. It is part of the microbiota of the oral, vaginal and intestinal mucous membranes of humans. Just like *L. salivarius, L. gasseri* is a well-known bacterial strain for probiotic applications.

The inventors found out that ***L. gasseri* expresses the following proteins** that surprisingly immune cross-react with anti-oxytocin antibodies and thus present an oxytocin-like effect:
- Elongation factor Tu having an amino acid sequence of SEQ ID NO:4,
- Glucose-6-phosphate isomerase having an amino acid sequence of SEQ ID NO:5,
- Phosphoglycerate kinase having an amino acid sequence of SEQ ID NO:6,
- Enolase 1 having an amino acid sequence of SEQ ID NO:7, and
- Enolase 2 having an amino acid sequence of SEQ ID NO:8,

In one specific embodiment, *L. gasseri* is the *L. gasseri* LMG P-29638 strain.

*Lactobacillus camelliae* is a lactic acid bacterium from the Lactobacillaceae family. *L. camelliae* was isolated from fermented tea leaves and has been reported as part of the microbiota of the oral, vaginal and intestinal mucous membranes of humans.

The inventors found out that ***L. camelliae* expresses the following proteins** that surprisingly immune cross-react with anti-oxytocin antibodies and thus present an oxytocin-like effect:
- Elongation factor Tu having an amino acid sequence of SEQ ID NO:9,
- Fructose tagatose bisphosphate aldolase having an amino acid sequence of SEQ ID NO:10,
- 2,5-didehydrogluconate reductase having an amino acid sequence of SEQ ID NO:11,
- Triosephosphate isomerase having an amino acid sequence of SEQ ID NO:12, and
- 2,3-bisphosphoglycerate-dependent phosphoglycerate mutase having an amino acid sequence of SEQ ID NO:13,

*L. plantarum* is a lactic acid bacterium from the Lactobacillaceae family that is found in a variety of niches. These niches include dairy, meat, and several vegetable fermentations, it is also found in the human gastrointestinal tract. *L. plantarum* is also a reported bacterial strain for probiotic applications.

The inventors found out that ***L. plantarum* expresses the following proteins** that surprisingly immune cross-react with anti-oxytocin antibodies and thus present an oxytocin-like effect:
- Elongation factor Tu having an amino acid sequence of SEQ ID NO: 14,
- GMP synthase having an amino acid sequence of SEQ ID NO: 15, and
- Glutamine-fructose-6-phosphate aminotransferase having an amino acid sequence of SEQ ID NO: 16.

*L. reuteri* is a lactic acid bacterium from the Lactobacillaceae family that naturally occurs in the gastric and intestinal microflora. *L. reuteri* has not been reported to be pathogenic; rather, it has been studied as a probiotic organism.

The inventors found out that ***L. reuteri* expresses the following proteins** that surprisingly immune cross-react with anti-oxytocin antibodies and thus present an oxytocin-like effect:
- Elongation factor Tu having an amino acid sequence of SEQ ID NO: 17, and
- Glucose-6-phosphate isomerase having an amino acid sequence of SEQ ID NO:18.

In one embodiment, the composition further comprises at least one *Bifidobacterium* strain. In one embodiment, the at least one *Bifidobacterium* strain is selected from the group consisting of *Bifidobacterium ruminantium, Bifidobacterium dentium, Bifidobacterium pseudolongum* subsp. *pseudologum, Bifidobacterium pseudolongum* subsp. *globosum* and/or *Bifidobacterium adolescentis.* In one embodiment, the at least one *Bifidobacterium* strain is *Bifidobacterium ruminantium.*

Several representatives of the genus Bifidobacterium have been reported as probiotic strains namely in view of their symbiotic nature with their host organism.

The inventors found out that ***B. dentium* expresses the following proteins** that surprisingly cross-react with anti-oxytocin antibodies and thus present an oxytocin-like effect:
- Phosphoglycerate kinase having an amino acid sequence of SEQ ID NO: 19, and
- Phosphoketolase having an amino acid sequence of SEQ ID NO:20.

The inventors found out that ***B. pseudolongum* subsp. *pseudolongum* expresses the following proteins** that surprisingly cross-react with anti-oxytocin antibodies and thus present an oxytocin-like effect:
- Elongation factor Tu having an amino acid sequence of SEQ ID NO:21, and
- Phosphoglycerate kinase having an amino acid sequence of SEQ ID NO:22.

The inventors found out that ***B. pseudolongum* subsp. *globosum* expresses the following proteins** that surprisingly cross-react with anti-oxytocin antibodies and thus present an oxytocin-like effect:
- Phosphoglycerate kinase having an amino acid sequence of SEQ ID NO:23, and
- Prolable phosphoketolase having an amino acid sequence of SEQ ID NO:24.

The inventors found out that ***B. ruminantium* expresses the following proteins** that surprisingly cross-react with anti-oxytocin antibodies and thus present an oxytocin-like effect:
- Phosphoketolase having an amino acid sequence of SEQ ID NO:25.

The inventors found out that ***B. adolescentis* expresses the following proteins** that surprisingly cross-react with anti-oxytocin antibodies and thus present an oxytocin-like effect:
- Elongation factor Tu having an amino acid sequence of SEQ ID NO:26.

The presence of oxytocin-like motifs in the above target proteins was searched by sequence alignment with the oxytocin amino acid sequence (SEQ ID NO: 27). Methods for comparing the identity of two or more sequences are well known in the art. The "needle" program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is, for example, available on the ebi.ac.uk world wide web site. The percentage of identity in accordance with the invention is preferably calculated using the EMBOSS: needle (global) program with a "Gap Open" parameter equal to 10.0, a "Gap Extend" parameter equal to 0.5, and a Blosum62 matrix.

It was surprisingly found that the following fragments not only present an homology with the oxytocin amino acid sequence (SEQ ID NO: 27) but also can activate the oxytocin receptor at a micromolar scale:
1) SEQ ID 28: H - DYVKNMITG - OH being the 87-95 position fragment of SEQ ID NO:1,
2) SEQ ID NO 29: H - KFVNDNELG- OH being the 13-21 position fragment of SEQ ID NO:2,
3) SEQ ID NO 30: H - KTVVWNGPMG - OH being the 317-326 position fragment of SEQ ID NO:3,
4) SEQ ID NO 31: H - ATSKNCPIA- OH being the 154-162 position fragment of SEQ ID NO:15,
5) SEQ ID NO 32: H - LTITNVPNS- OH being the 371-379 position fragment of SEQ ID NO:16,
6) SEQ ID NO 33: H - QYIQEGR- OH being the 313-319 position fragment of SEQ ID NO:5,
7) SEQ ID NO 34: H - KQFVHENELG- OH being the 12-21 position fragment of SEQ ID NO:18,
8) SEQ ID NO 35: H - DYIKNMITG - OH being the 88-96 position fragment of SEQ ID NO:4,
9) SEQ ID NO 36: H - VDIQEFMIMPVG - OH being the 160-171 position fragment of SEQ ID NO:8,
10) SEQ ID NO 37: H - SYFYNKEDG - OH being the 246-254 position fragment of SEQ ID NO:7,
11) SEQ ID NO 38: H-MFQAARKG-OH being the 1-8 position fragment of SEQ ID NO:10,
12) SEQ ID NO 39: H-AYSPLGTGK-OH being the 202-210 position fragment of SEQ ID NO:11,
13) SEQ ID NO 40: H-CYFEDAGA-OH being the 61-68 position fragment of SEQ ID NO:12,
14) SEQ ID NO 41: H-KYIENISDE-OH being the 191-199 position fragment of SEQ ID NO:13,
15) SEQ ID NO 42: H-IYLRSNPLMK-OH being the 40-49 position fragment of SEQ ID NO:20,
16) SEQ ID NO 43: H-GFDRDCPVV-OH being the 164-172 position fragment of SEQ ID NO:21,
17) SEQ ID NO 44: H-DFVKNMITG-OH being the 89-97 position fragment of SEQ ID NO:21.

In one embodiment, the composition comprises a mixture of at least two strains that express a peptide presenting from at least 80%, from at least 85%, from at least 90%, from at least 95%, or 100% identity with at least two, preferably at least three of the peptides selected from the group consisting of SEQ ID NO: 28, SEQ ID NO: 29, SEQ ID NO: 30, SEQ ID NO: 33, SEQ ID NO: 35, SEQ ID NO: 36 and SEQ ID NO: 37. In one embodiment, the composition comprises a mixture of at least two strains that express a peptide presenting from at least 80%, from at least 85%, from at least 90%, from at least 95%, or 100% identity with at least two, preferably at least three of the peptides selected from the group consisting of SEQ ID NO: 28, SEQ ID NO: 29, SEQ ID NO: 30 and SEQ ID NO: 33. In a typical embodiment, the composition comprises a mixture of at least two strains selected from *Lactobacillus salivarius* and *Lactobacillus gasseri.* In a specific embodiment, the composition comprises a mixture of at least two strains selected from *L. salivarius* DSM 16530 strain and *L. gasseri* LMG P-29638 strain.

In another embodiment, the composition comprises a mixture of at least two, preferably at least three strains that express a peptide presenting from at least 80%, from at least 85%, from at least 90%, from at least 95%, or 100% identity with at least two, preferably at least three of the peptides selected from the group consisting of SEQ ID NO: 28, SEQ ID NO: 29, SEQ ID NO: 30, SEQ ID NO: 38, SEQ ID NO: 39, SEQ ID NO: 40, SEQ ID NO: 41 and SEQ ID NO: 42. In one embodiment, the at least two, preferably at least three of the peptides selected from the group consisting of SEQ ID NO: 28, SEQ ID NO: 29, SEQ ID NO: 30, SEQ ID NO: 41 and SEQ ID NO: 42. In a typical embodiment, the composition comprises a mixture of at least three strains selected from *Lactobacillus salivarius, Lactobacillus camelliae* and *Bifidobacterium ruminantium.*

The amount of each strain in the composition of the invention may vary in a wide range. The amount of each strain in the composition of the invention may be comprised between 10² and 10¹³ CFU, preferably between 10⁵ and 10¹² CFU, in particular between 10⁹ and 10¹⁰ CFU. In some embodiments, the amount of each strain in the composition is about 10², 10³, 10⁴, 10⁵, 10⁶, 10⁷, 10⁸, 10⁹, 10¹⁰, 10¹¹, 10¹² or 10¹³ CFU. One of ordinary skill in the art is able to determine, depending on the strains, on the other components of the composition, and on the desired effect of the composition, the appropriate amount of each strain in the composition.

The total amount of strains in the composition of the invention may vary in a wide range. The total amount of strains in the composition of the invention may be comprised between 10⁴ and 10¹⁵ CFU, preferably between 10⁶ and 10¹³ CFU, in particular between 10⁹ and 10¹¹ CFU. In some embodiments, the amount of each strain in the composition is about 10⁴, 10⁵, 10⁶, 10⁷, 10⁸, 10⁹, 10¹⁰, 10¹¹, 10¹², 10¹³, 10¹⁴ or 10¹⁵ CFU. One of ordinary skill in the art is able to determine, depending on the strains, on the other components of the composition, and on the desired effect of the composition, the appropriate total amount of strains in the composition.

The relative amount of one strain to the other strain(s) in the composition may vary in a wide range, depending, among others, on the number of different strains in the composition. In some embodiments, each strain is present in an equal amount. In such an embodiment, if the composition of the invention comprises exactly two strains, each strain is present in an amount that is half the total amount of strains. In another such embodiment, if the composition of the invention comprises exactly three strains, each strain is present in an amount that is a third of the total amount of strains. In other embodiments, the amount of the different strains of the composition is not the same.

In one embodiment, the composition comprises 10¹⁰ CFU of *Lactobacillus salivarius* and 10¹⁰ CFU of *Lactobacillus gasseri.* Preferably, according to this embodiment, *Lactobacillus salivarius* and *Lactobacillus gasseri* are the only active strains of the composition, preferably the only strains of the composition.

In another embodiment, the composition comprises a total amount of strains of 10¹⁰ CFU, and comprises *Lactobacillus salivarius Lactobacillus camelliae* and *Bifidobacterium ruminantium,* each strain representing a third of the total amount of strains. Preferably, according to this embodiment, *Lactobacillus salivarius Lactobacillus camelliae* and *Bifidobacterium ruminantium* are the only active strains of the composition, preferably the only strains of the composition.

The composition of the invention may further comprise any suitable other components known in the art.

In an embodiment, the composition according to the invention further comprises at least one plant or plant extract, preferably selected from the group consisting of *Rhodiola rosea, Withania somnifera, Melissa officinalis, Crocus sativus* and *Hypericum perforatum.* In one embodiment, the at least one plant or plant extract is *Rhodiola rosea.*

In an embodiment, the composition according to the invention further comprises at least one mineral supplement, preferably a magnesium supplement. The magnesium supplement may be selected from a magnesium salt or magnesium bound to an ammo acid. In one embodiment, the magnesium salt is selected from the group consisting of magnesium carbonate, magnesium oxide, magnesium acetate, magnesium ascorbate, magnesium citrate, magnesium gluconate, magnesium lactate, magnesium malate, magnesium pyrrolidone carboxylate, magnesium taurate, and magnesium threonate.

In some embodiments, the composition of the invention is a pharmaceutical composition and/or a nutraceutical composition.

Pharmaceutical and nutraceutical compositions according to the invention differ mainly in their intended use. Main features that may vary between pharmaceutical and nutraceutical compositions according to the invention are: the used strains combination, the form of the composition, the excipients and/or the dosing of each strain in the composition.

The composition of the invention may further comprise at least one excipient, preferably at least one pharmaceutically and/or nutraceutically acceptable excipient.

The composition of the invention, in particular the pharmaceutical and/or nutraceutical composition of the invention, may be formulated to be suitable for any type of administration. For instance, the composition of the invention, in particular the therapeutic and/or nutraceutical composition of the invention, may be formulated to be suitable for oral, rectal, percutaneous, permucosal and/or intravenous administration, and/or for administration by injection.

One skilled in the art is able to select the appropriate excipients and/or vehicles for preparing, from the strains, a composition that is suitable for any type of administration. A composition may thus be presented in the form of injectable solutions or suspensions or multi-dose bottles, in the form of plain or coated tablets, sugarcoated tablets, wafer capsules, gel capsules, pills, cachets, powders, suppositories or rectal capsules, solutions or suspensions such as for example solutions or suspensions for intranasal administration, for percutaneous use in a polar solvent, or for permucous use.

Examples of excipients that are suitable for such administrations are cellulose derivatives, microcrystalline cellulose derivatives, alkaline-earth metal carbonates, magnesium phosphate, starches, modified starches and lactose for the solid forms.

For rectal use, cocoa butter or polyethylene glycol stearates are the preferred excipients.

For parenteral and/or intranasal use, water, aqueous solutions, physiological saline and isotonic solutions are the vehicles that are the most suitable.

In some embodiments, the composition of the invention, in particular the therapeutic and/or nutraceutical composition of the invention, is an oral composition, in particular an oral therapeutic and/or nutraceutical composition. An oral composition is a composition that is suitable for oral administration. Suitable forms for oral administration include uncoated or coated tablets, sugar-coated tablets, capsules, gelatin capsules, pills, cachets, powders.

Suitable excipients for formulation in the form of a tablet or capsule include oral, non-toxic, pharmaceutically acceptable inert carriers such as ethanol, glycerol, water, and the like. Suitable binders, lubricants, disintegrants, and color developers may also be included in the mixture, if desired or desired. Suitable binders include, but are not limited to, natural sugars such as starch, gelatin, glucose or beta-lactose, corn sweeteners, natural and synthetic gums such as acacia, track can or sodium oleate, sodium stearate, magnesium stearate, sodium benzoate, sodium acetate, sodium chloride, and the like. Disintegrants include, but are not limited to, starch, methylcellulose, agar, bentonite, xanthan gum, and the like. Pharmaceutical carriers acceptable for compositions formulated as liquid solutions include, but are not limited to, saline, sterile water, Ringer's solution, buffered saline, albumin injection solutions, Dextrose solution, maltodextrin solution, glycerol, ethanol, and one or more of these components may be mixed and used, and other conventional additives such as antioxidants, buffers, bacteriostats, and the like may be added as necessary. Diluents, dispersants, surfactants, binders and lubricants may also be added to formulate into injectable formulations, pills, capsules, granules or tablets such as aqueous solutions, suspensions, emulsions and the like.

In some embodiments, the composition according to the invention is a nutraceutical composition. The nutraceutical composition according to the invention may be in particular a food composition.

The food composition according to the present invention can be added to various foods. Foods to which the composition of the present invention can be added include, for example, beverages, vitamin complexes, dietary supplements, and the like.

The food compositions of the present invention may comprise ingredients conventionally added in food preparation and include, for example, proteins, carbohydrates, fats, nutrients, flavoring agents and flavoring agents. Examples of the carbohydrates described above are monosaccharides such as glucose, fructose, and the like; disaccharides such as maltose, sucrose, oligosaccharide, and the like; and polysaccharides such as conventional sugars such as dextrin, cyclodextrin, and the like, and sugar alcohols such as xylitol, sorbitol, erythritol, and the like. Natural flavors (taumatin, stevia extracts (e.g., rebaudioside a, glycyrrhizin, etc.)) and synthetic flavors (saccharin, aspartame, etc.) can be used as flavors. For example, when the food composition of the present invention is prepared from a drink and a beverage, citric acid, liquid fructose, sugar, glucose, acetic acid, malic acid, fruit juice, and various plant extracts may be further included.

In some embodiments of the composition according to the invention, the strains are encapsulated in order to be protected against the stomach. Accordingly, in some embodiments, the strains are in an encapsulated form so as significantly to improve their survival time. In such a case, the presence of a capsule may in particular delay or prevent the degradation of the microorganisms in the gastrointestinal tract. It will be appreciated that the compositions of the present embodiments can be encapsulated into an enterically-coated, time-released capsule or tablet. The enteric coating allows the capsule/tablet to remain intact (i.e., undissolved) as it passes through the gastrointestinal tract, until such time as it reaches the small intestine. Methods of encapsulating live bacterial cells are well known in the art (see, e.g., U.S. patents to General Mills Inc. such as U.S. Pat. No. 6,723,358). For example, micro-encapsulation with alginate and Hi-Maize^{™} starch followed by freeze-drying has been proved successful in prolonging shelf-life of bacterial cells in dairy products [see, e.g., Kailasapathy et al. Curr Issues Intest Microbiol. 2002 September; 3(2):39-48]. Alternatively, encapsulation can be done with glucomannane fibers such as those extracted from Amorphophallus konjac. Alternatively, entrapment of viable probiotic in sesame oil emulsions may also be used [see, e.g., Hou et al. J. Dairy Sci. 86:424-428]. In some embodiments, agents for enteric coatings are preferably methacrylic acid- alkyl acrylate copolymers, such as Eudragit^{®} polymers. Poly(meth)acrylates have proven particularly suitable as coating materials. EUDRAGIT^{®} is the trade name for copolymers derived from esters of acrylic and methacrylic acid, whose properties are determined by functional groups. The individual EUDRAGIT^{®} grades differ in their proportion of neutral, alkaline or acid groups and thus in terms of physicochemical properties. The skillful use and combination of different EUDRAGIT^{®} polymers offers ideal solutions for controlled drug release in various pharmaceutical and technical applications. EUDRAGIT^{®} provides functional films for sustained-release tablet and pellet coatings. The polymers are described in international pharmacopeias such as Ph.Eur., USP/NF, DMF and JPE. EUDRAGIT^{®} polymers can provide the following possibilities for controlled drug release: gastrointestinal tract targeting (gastroresistance, release in the colon), protective coatings (taste and odor masking, protection against moisture) and delayed drug release (sustained-release formulations). EUDRAGIT^{®} polymers are available in a wide range of different concentrations and physical forms, including aqueous solutions, aqueous dispersion, organic solutions, and solid substances. The pharmaceutical properties of EUDRAGIT^{®} polymers are determined by the chemical properties of their functional groups. A distinction is made between:
- poly(meth)acrylates, soluble in digestive fluids (by salt formation) EUDRAGIT^{®} L (Methacrylic acid copolymer), S (Methacrylic acid copolymer), FS and E (basic butylated methacrylate copolymer) polymers with acidic or alkaline groups enable pH-dependent release of the active ingredient. Applications: from simple taste masking via resistance solely to gastric fluid, to controlled drug release in all sections of the intestine.
- poly(meth)acrylates, insoluble in digestive fluids: EUDRAGIT^{®} RL and RS (ammonio methacrylate copolymers) polymers with alkaline and EUDRAGIT^{®} NE polymers with neutral groups enable controlled time release of the active by pH-independent swelling.

Enteric EUDRAGIT^{®} coatings provide protection against drug release in the stomach and enable controlled release in the intestine. The dominant criterion for release is the pH-dependent dissolution of the coating, which takes place in a certain section of the intestine (pH 5 to over 7) rather than in the stomach (pH 1-5). For these applications, anionic EUDRAGIT^{®} grades containing carboxyl groups can be mixed with each other. This makes it possible to finely adjust the dissolution pH, and thus to define the drug release site in the intestine. EUDRAGIT^{®} L and S grades are suitable for enteric coatings. EUDRAGIT^{®} FS 30 D (aqueous dispersion of an anionic copolymer based on methyl acrylate, methyl methacrylate and methacrylic acid) is specifically used for controlled release in the colon.

In some embodiments, the food composition of the present invention is selected from complete food compositions, food supplements, and the like. The composition of the present invention may be used as a food ingredient and/or feed ingredient.

The food ingredient may be in the form of a solution or as a solid - depending on the use and/or the mode of application and/or the mode of administration.

The nutraceutical composition of the present invention may be solid, semi-solid or liquid. It may be in the form of a food product or food supplement, e.g. in the form of tablets, gels, powders, capsules, drinks, bars, etc. For example, the composition may be in the form of a powder packed in a sachet which can be dissolved in water, fruit juice, milk or another beverage.

In some embodiments, the drink is a functional drink or a therapeutic drink, a thirst-quencher or an ordinary drink. By way of example, the composition of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.

In some embodiments, the composition of the present invention is used with yoghurt production, such as fermented yoghurt drink, yoghurt, drinking yoghurt, cheese, fermented cream, milk based desserts and others. Suitably, the composition can be further used as an ingredient in one or more of cheese applications, meat applications, or applications comprising protective cultures.

In some embodiments, the food composition of the present invention is suitable for preparing meal replacement product.

The food composition of the present invention typically comprises carriers or vehicles. Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

In some embodiments, the food composition of the present invention comprises an amount of dietary fibers. Dietary fibers pass through the small intestine undigested by enzymes and function as a natural bulking agent and laxative. Dietary fiber may be soluble or insoluble and in general a blend of the two types is preferred. Suitable sources of dietary fibers include soy, pea, oat, pectin, guar gum, gum Arabic, fructooligosaccharides, galacto-oligosaccharides, sialyl-lactose and oligosaccharides derived from animal milks. In some embodiments, the dietary fiber is selected among mannans. Mannans (such as glucomannans and galactomannans), such as guar gum, locust bean gum, konjac, and xanthan gum, are present in some plant cell walls. The glucomannans are generally comprised of (1-4)-(β-linked glucose and mannose units, while the galactomannans are generally comprised of a (1-4)-β-mannan backbone substituted with single units of (1-6)-α-galactose. Many endospermic legumes, such as guar and locust bean, contain galactomannans in the endosperm during seed development. Glucomannans have also been found as a minor component of cereal grains.

In some embodiments, the food composition of the present invention contains minerals and micronutrients such as trace elements and vitamins in accordance with the recommendations of Government bodies such as the USRDA. For example, the composition may contain per daily dose one or more of the following micronutrients in the ranges given: 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, and/or 3 to 10 µg Vitamin E.

In some embodiments, the composition of the present invention contains emulsifiers. Examples of food grade emulsifiers typically include diacetyl tartaric acid esters of mono- and di-glycerides, lecithin and mono- and di-glycerides. Similarly, suitable salts and stabilizers may be included.

In some embodiments, the composition of the present invention contains protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The composition may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

In one embodiment, the composition, the pharmaceutical composition or the food composition of the invention is formulated in an oral dosage form selected from enterically-coated tablets and enterically-coated capsules, wherein the enteric-coating is a mixture comprising hydroxypropyl methyl cellulose and gellan gum. In one preferred embodiment, the oral dosage form is an enterically-coated capsule, wherein the enteric-coating is a mixture comprising hydroxypropyl methyl cellulose and gellan gum. In one embodiment, the enteric coating is the capsule itself. In one embodiment, the enteric coating comprises from 85 to 95 % hydroxypropyl methyl cellulose and from 5 to 15% gellan gum (w/w) in weight relative to the enteric-coating or the capsule weight. In one embodiment, the enteric coating comprises about 95 % hydroxypropyl methyl cellulose and about 5% gellan gum (w/w) in weight relative to the enteric-coating or the capsule weight. In one specific embodiment, the enteric-coating is a DRcaps ^{™} capsule commercialized by Capsugel^{®}.

In a last aspect, the invention relates to a blister, or preferably a container comprising the composition, the pharmaceutical composition, the food composition or at least one oral dosage form as previously described. Typically, the blister or preferably the container is moisture-tight. In one embodiment, the moisture-tight container comprises (a) a container body having a base and a sidewall extending therefrom, the container body defining an interior, the container body further having an opening leading to the interior and a lip surrounding the opening; (b) a lid being movable with respect to the container between a closed position in which the lid covers the opening so as to create a moisture tight seal with the container body and an open position in which the opening is exposed; (c) at least a first seal and a second seal, the first seal being formed by mating thermoplastic- to-thermoplastic sealing surfaces of the lid and the container body respectively, the first seal optionally including an undercut of the container body relative to a central axis of the container body or a lip seal member extending downward from the lid, the second seal being formed by mating elastomer-to-thermoplastic sealing surfaces, wherein the elastomer-to-thermoplastic sealing surfaces includes an elastomer formed in the lid or on the container body, optionally with multi-shot injection molding, wherein the thermoplastic is incompressible and the elastomer is compressible and optionally resilient; and optionally (d) an insert secured within the interior of the container body, the insert comprising a base material and a desiccant, wherein the base material provides structure to the insert and is optionally a polymer, the insert having an insert opening leading to an interior compartment configured for housing products. Advantageously, the container, when in the closed position, has a moisture vapor transmission rate, at ambient conditions of 30°C and 75% relative humidity (RH), of less than 500 µg/day, less than 400 µg/day, less than 350 µg/day, less than 325 µg/day, less than 300 µg/day, from 150 µg/day to 300 µg/day, from 175 µg/day to 285 µg/day. In one specific embodiment, the moisture tight container is an Aptar CSP Technologies container such as for example Activ-vial^{™}.

### Therapeutic and/or nutraceutical use of the compositions

The present invention further relates to a composition according to the invention, preferably a pharmaceutical or nutraceutical composition according to the invention, for use in altered mood disorders, depression, autism and other stress-related disorders.

In particular, the present invention relates to a composition for use in the treatment of mood disorders such as anxiety, depression and/or autism; said composition comprising *Lactobacillus salivarius* strain and least one second *Lactobacillus* strain, and optionally at least one *Bifidobacterium* strain. Preferably, the composition for use is a pharmaceutical composition according to the invention as detailed above. All embodiments listed above regarding the pharmaceutical compositions of the invention apply *mutatis mutandis* to the composition for use according to the invention.

The present invention further relates to the use of a composition comprising *Lactobacillus salivarius* strain and least one second *Lactobacillus* strain, and optionally at least one *Bifidobacterium* strain in the manufacture of a medicament for the treatment of mood disorders such as anxiety, depression and/or autism.

The present invention further relates to method for the treatment of mood disorders such as anxiety, depression and/or autism, comprising administering to a subject in need thereof a therapeutically and/or nutraceutically effective amount of a composition comprising *Lactobacillus salivarius* strain and least one second *Lactobacillus* strain, and optionally at least one *Bifidobacterium* strain.

Mood disorders include psychological responses to stresses to which a subject is exposed. Mood disorders may include for instance depression, anxiety, fatigue, mood changes, stress, schizophrenia and/or bipolar disorder. Autism is a developmental disorder characterized by difficulties with social interaction and communication, and by restricted and repetitive behavior. Autism is also encompassed among the pathologies and/or disorders that may be treated according to the present invention.

Depression refers to psychological states of aggressed mood characterized by sensations of sadness, satiety and left savings. That is, developing major depressive disorder via normal emotion, "aggressive", dysthymic disorder. Major symptoms include changes in mouth taste, body weight or sleep pattern, psychomotor agitation or retardation, accident ability, reduction in concentration or crystallinity, lack of activity and fatigue, emotion of unvaluable, A major depressive episode, which is a period of time that indicates the loss of interest or enjoyment in a routine mood or majority of activities, combined with some of frequent dead or suicidal vigour, trial plans or attempts.

The compositions to be used according to the invention are preferably therapeutical and/or nutraceutical compositions according to the invention.

The present invention further relates to the use of a nutraceutical composition according to the invention in the management of mood disorders. The nutraceutical composition according to the invention is preferably used for promoting, maintaining and/or improving comfort or for alleviating and/or preventing a discomfort in a subject suffering from at least one mood disorder or any symptom thereof.

In some embodiments, the invention relates to the use of a nutraceutical composition according to the invention for alleviating stress or anxiety, or any symptom thereof. In some embodiments, the nutraceutical composition according to the invention is used for improving the mood and/or for relieving the every-day stress of a healthy subj ect.

The posology may vary within a wide range depending on the therapeutic and/or nutraceutical indication and the route of administration, and also the general health, age, sex, and body weight of the individual. The skilled artisan will be able to determine appropriate dosages depending on these and other factors.

For example, when the composition according to the invention is administered as a pharmaceutical composition, it may typically comprise a total amount of strains ranging from 10⁵ to 10¹⁵ CFU per gram of therapeutical composition.

When the composition according to the invention is administered as a nutraceutical composition, for instance as a food product, it may typically comprise a total amount of strains ranging from 10³ to 10¹² CFU per gram of nutraceutical composition, preferably per gram of food product.

In some embodiments, the administration of the composition according to the invention, preferably of the pharmaceutical and/or nutraceutical composition according to the invention, is repeated, for example, 2 to 3 times a day, for one day or more and generally for a sustained period of at least 4 days, or even 4 to 15 weeks, with, where appropriate, one or more periods of interruption.

In some embodiments, the composition according to the invention, preferably of the pharmaceutical and/or nutraceutical composition according to the invention, is administered simultaneously or sequentially to a meal of the subject. In some embodiments, the composition according to the invention is administered prior to the meal of the subject.

In some embodiments, the pharmaceutical compositions according to the invention are administered in combination with at least another active agent, preferably another active agent in the treatment of mood disorders, for instance an anti-depressive agent.

The combination of the pharmaceutical composition of the invention with at least one other active agent may be simultaneous, sequential or separate.

In one embodiment, the at least one other active agent may be at least one selective serotonin reuptake inhibitor and/or at least one serotonin-norepinephrine reuptake inhibitor. In one embodiment, the at least one selective serotonin reuptake inhibitor is selected from the group consisting of citalopram, escitalopram, fluoxetine, fluvoxamine, paroxetine, and sertraline. In one embodiment, the at least one serotonin-norepinephrine reuptake inhibitor is selected from the group consisting of atomoxetine, desvenlafaxine, duloxetine, levomilnacipran, milnacipran and venlafaxine.

### Peptide compositions

The inventors found that the Lactobacillus and/or Bifidobacterium strains described above comprise at least one peptide of SEQ ID NO: 1 to SEQ ID NO:26 that cross-reacts with oxytocin antibodies and are therefore conformational mimetics of oxytocin. The invention finally relates to a composition comprising at least one bioactive peptide that is able to cross-react with anti-oxytocin antibodies and/or presents an oxytocin-like effect.

The at least one bioactive peptide may be selected from the group consisting of any one of peptides of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17; SEQ ID NO:18; SEQ ID NO:19; SEQ ID NO:20; SEQ ID NO:21; SEQ ID NO:22; SEQ ID NO:23; SEQ ID NO:24; SEQ ID NO:25, and SEQ ID NO:26.

The at least one bioactive peptide may be selected from a peptide presenting at least 99%, at least 98%, at least 97%, at least 96%, at least 95%, at least 94%, at least 93%, at least 92%, at least 91% or at least 90% identity with any one of the above peptides of SEQ ID NO:1 to SEQ ID NO:26. The at least one bioactive peptide may be selected from a peptide presenting from at least 80%, from at least 85%, from at least 90 or from at least 95% identity with any one of the above peptides of SEQ ID NO:1 to SEQ ID NO:26.

To the inventors surprise the above peptides are not only conformational mimetics of oxytocin but also comprise segments that directly activate the oxytocin-receptor. Therefore, the at least one bioactive peptide may be selected from a peptide fragment of any one of the above peptides of SEQ ID NO: 1 to SEQ ID NO:26.

Typically, the peptide fragment is selected from the group consisting of the peptides SEQ ID NO:28 to SEQ ID NO: 44. In one embodiment, the peptide is selected from the group consisting of any one of the peptides SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43 and /or SEQ ID NO:44. In one embodiment, the peptide is selected from a peptide presenting from at least 80%, from at least 85%, from at least 90 or from at least 95% identity with any one of the above peptides of SEQ ID NO:28 to SEQ ID NO:44.

According to a first embodiment, the peptide is selected from a peptide presenting from at least 80%, from at least 85%, from at least 90 from at least 95%, or 100% identity with any one of the following peptides:
1) SEQ ID 28: H - DYVKNMITG - OH,
2) SEQ ID NO 29: H - KFVNDNELG- OH,
3) SEQ ID NO 30: H - KTVVWNGPMG - OH,
4) SEQ ID NO 31: H - ATSKNCPIA- OH,
5) SEQ ID NO 32: H - LTITNVPNS- OH,
6) SEQ ID NO 33: H - QYIQEGR- OH,
7) SEQ ID NO 34: H - KQFVHENELG- OH,
8) SEQ ID NO 35: H - DYIKNMITG - OH,
9) SEQ ID NO 36: H - VDIQEFMIMPVG - OH,
10) SEQ ID NO 37: H - SYFYNKEDG - OH,
11) SEQ ID NO 38: H-MFQAARKG-OH,
12) SEQ ID NO 39: H-AYSPLGTGK-OH,
13) SEQ ID NO 40: H-CYFEDAGA-OH,
14) SEQ ID NO 41: H-KYIENISDE-OH,
15) SEQ ID NO 42: H-IYLRSNPLMK-OH,
16) SEQ ID NO 43: H-GFDRDCPVV-OH,
17) SEQ ID NO 44: H-DFVKNMITG-OH.

According to a second embodiment, the peptide is selected from a peptide presenting from at least 80%, from at least 85%, from at least 90 from at least 95%, or 100% identity with any one of the following peptides:
1) SEQ ID 28: H - DYVKNMITG - OH,
2) SEQ ID NO 29: H - KFVNDNELG- OH,
3) SEQ ID NO 30: H - KTVVWNGPMG - OH,
4) SEQ ID NO 31: H - ATSKNCPIA- OH,
5) SEQ ID NO 32: H - LTITNVPNS- OH,
6) SEQ ID NO 33: H - QYIQEGR- OH,
7) SEQ ID NO 34: H - KQFVHENELG- OH,
8) SEQ ID NO 35: H - DYIKNMITG - OH,
9) SEQ ID NO 41: H-KYIENISDE-OH,
10) SEQ ID NO 44: H-DFVKNMITG-OH.

The composition comprising at least one bioactive peptide according to the invention may comprise all the same components as descried above for the compositions comprising the strain combinations, apart from the strains themselves.

The composition comprising at least one bioactive peptide according to the invention may be used as disclosed above for nutraceutical and/or pharmaceutical applications.

In particular, the invention relates to a bioactive peptide or a pharmaceutical composition comprising at least one bioactive peptide according to the invention for use in the treatment of at least one neuropsychiatric disorder, such as anxiety, depression and/or autism. In one specific embodiment, the bioactive peptide or the pharmaceutical composition according to the invention is for use in the treatment of depression.

The invention also relates to the use of a bioactive peptide or a nutraceutical composition comprising at least one bioactive peptide according to the invention in the management of mood disorders. The bioactive peptide or a nutraceutical composition comprising at least one bioactive peptide according to the invention is preferably used for promoting, maintaining and/or improving comfort or for alleviating and/or preventing a discomfort in a subject suffering from at least one mood disorder or any symptom thereof.

In some embodiments, the invention relates to the use of a bioactive peptide or a nutraceutical composition comprising at least one bioactive peptide according to the invention for alleviating stress or anxiety, or any symptom thereof. In some embodiments, the bioactive peptide or the nutraceutical composition comprising at least one bioactive peptide according to the invention is used for improving the mood and/or for relieving the every-day stress of a healthy subject.

All embodiments and features disclosed above for the compositions comprising strain combinations according to the invention and for their therapeutical and/or nutraceutical use apply similarly to the bioactive peptide or composition comprising at least one bioactive peptide according to the invention.

### BRIEF DESCRIPTION OF THE DRAWINGS

**Figure 1** is a histogram showing the effect of Mix 1 treatment in O-maze test. ** for p<0.01 with two-way ANOVA analysis.
**Figure 2** is a histogram showing the effect of Mix 1 treatment in water wheel test. * for p<0.05 in Student's test.
**Figure 3** is a histogram showing the effect of Mix 1 treatment on mobility in the open field test. "*" for p<0.05 and "**" for p<0.01 with two-way ANOVA analysis.
**Figure 4** is a histogram showing the effect of Mix 1 treatment on anxiety in the open field test. The effect is measured by the percentage of distance moved in the center (left) or the percentage of time in spent in the center of the open field (right) which as considered anxiogenic. They were calculated before and after 10 days of gavage in control group (white) and Lactobacilli treated group (black) taking total distance moved or total 1h time as references, respectively. (n=8 per group) "*" for p<0.05 with paired t-test analysis.
**Figure 5** is a graph showing the effect of Mix 1 in the tail suspension test. Cumulative immobility (left) and cumulative energy exerted to escape (right) for 6 minutes in control (withe circle) and treated (black square) groups (n=7 per group). "*" for p<0.05 and "**" for p<0.01 two-way ANOVA analysis.
**Figure 6** is a graph showing the effect of Mix 1 in forced swim test. Immobility time was measured for 6 min in control (white circle) and treated (black square) groups (n=8 per group). "*" for p<0.05 with two-way ANOVA analysis.
**Figure 7** is a graph showing the effect of individual bacterial strains and of Mix 2 on immobility in FST. Immobility time was calculated for 6 minutes in control (black) and treated (color) groups (n=8 per group). "*" for p<0.05 with one-way ANOVA analysis.

### EXAMPLES

The present invention is further illustrated by the following examples.

### Example 1: Screening of bacterial strains - Proteomic analysis

The present study aims at screening Lactobacilli and Bidobacteria for their capacity of their components to present oxytocin-like effects.

### Materials and Methods

In this study, 14 bacteria-extracted proteomes were analyzed: (i) from 5 Bifidobacterium species grown in anaerobe conditions, (ii) from 5 Lactobacillus species grown in anaerobe culture conditions and (iii) from 4 aerotolerant Lactobacillus species grown in aerobe conditions. Each proteome analysis was performed in triplicate and was further separated in 3 fractions i.e. soluble (S), membrane (M), and residual (R) resulting in 9 samples / proteome). Each fraction was initially screened by western blot (WB) for the presence OT-like reactivity to validate it for further 2D-SDS-PAGE analysis. The WB revealed OT-like immunoreactive bands in most but not all samples (data not shown). Finally, all fractions have been analyzed using 2D gel technique and OT immunodetection. 2D gel electrophoresis allowed to improve visibility of the less abundant proteins in a complex sample. Thereby, all samples were analyzed at least once with this method to scan optimally each proteome in the search for OT mimicry. By this way, some samples for which no OT-immunoreactivity had been identified by 1D-western blot, showed positive results by 2D-immunoblotting. Samples exhibited positive immunoreactivity with OT IgG were analyzed in duplicate. The specificity for OT-like staining was controlled by pre-adsorption of OT-IgG with OT peptide.

All protein spots in silver nitrate staining gels corresponding to OT-like spots in the membrane were excised and analyzed by mass spectrometry

### Results

The proteomic analysis revealed 44 target proteins: 27 in Lactobacillus and 17 in Bifidobacterium, variably distributed in all samples. Among them, 7 were common to both genus: phosphoglycerate kinase (PGK), elongation factor thermo unstable (EFTU), enolase and 3 chaperone proteins. Conversely, some target proteins were specific to the genus or to a strain.

Target proteins were first selected based on their alignment score with the OT sequence. Then, they were sorted according to their recurrence (i) between all samples for common proteins, (ii) between strains for a genus-specific protein and (iii) between replicates for a strain-specific protein. The 60 kDa chaperonin protein was eliminated from 7 common target proteins despite its high recurrency between samples because the molecular weight of the spot did not always match, which may due to contamination of the target spot.

The PGK (phosphoglycerate kinase), EFTU (elongation factor tu) and ENO (enolase) have been found to represent common proteins group. In fact, PGK was identified in 5 strains (2 Lactobacillus and 3 Bifidobacterium), EFTU in 7 strains (5 Lactobacillus and 2 Bifidobacterium) and ENO in 5 strains (2 Lactobacillus and 3 Bifidobacterium). The glucose-6-phosphate isomerase (G6PI) was identified in LS (*L. salivarius*), LG (*L. gasseri*) and LR (*L. reuteri*) strains *i.e.* was Lactobacillus genus specific. Bifidobacterium group was represented by a phosphoketolase protein identified in BR (*B. ruminantium*), BD *(B. dentis)* and BPG *(B pseudolongum subsp. globosum)* strains. Proteomes obtained from LP *(L. plantarum)* and LC *(L. camelliae* were different from the other strains. Proteomic analysis revealed respectively 2 and 4 representative targets of these 2 distinctive strains. Although the common and Lactobacillus-specific targets are highly conserved in LP and LC, they were not or poorly identified in these strains. Similarly, the GMP synthase (GUAA) and the aminotransferase (GLMS) which represented LP, as well as the aldolase, the reductase, the isomerase and the mutase found in LC, were not identified in other Lactobacilli. These 6 proteins were identified as LP or LC-specific targets (Table 1).

Based on the results on OT-like protein identification, 17 proteins were selected for the synthesis of target peptide fragments. These peptides corresponded to the part of the target sequences which aligned with the OT sequence. Altogether, 17 potential OT-like peptides derived from the proteomic analysis have been selected and synthesized (Table 1).

**Table 1 showing the OT-like peptide fragments derived from the proteomic analysis**

| **#Id.** | **Protein name** | **Peptide Sequence** | **Position** | **SEQ ID NO:** | **Bacterial origin** |
|---|---|---|---|---|---|
| 1 | Elongation factor Tu | H - DYVKNMITG - OH | 87-95 | **28** | LS, LR, LP, LC |
| 2 | Glucose-6-phosphate isomerase | H - KFVNDNELG- OH | 13-21 | **29** | LS |
| 3 | Phosphoglycerate kinase | H - KTVVWNGPMG - OH | 317-326 | **30** | LG, LS, BD, BPP, BPG |
| 4 | GMP synthase [glutamine-hydrolyzing] | H - ATSKNCPIA- OH | 154-162 | **31** | LP |
| 5 | Glutamine-- fructose-6-phosphate aminotransferase [isomerizing] | H - LTITNVPNS- OH | 371-379 | **32** | LP |
| 6 | Glucose-6-phosphate isomerase | H - QYIQEGR- OH | 313-319 | **33** | LG |
| 7 | Glucose-6-phosphate isomerase | H - KQFVHENELG- OH | 12-21 | **34** | LR |
| 8 | Elongation factor Tu | H - DYIKNMITG - OH | 88-96 | **35** | LG |
| 9 | Enolase 2 | H - VDIQEFMIMPVG - OH | 160-171 | **36** | LG |
| 10 | Enolase | H - SYFYNKEDG - OH | 246-254 | **37** | LG |
| 11 | Fructose tagatose bisphosphate aldolase | H-MFQAARKG-OH | 1-8 | **38** | LC |
| 12 | 2,5-didehydrogluconate reductase | H-AYSPLGTGK-OH | 202-210 | **39** | LC |
| 13 | Triosephosphate isomerase | H-CYFEDAGA-OH | 61-68 | **40** | LC |
| 14 | 2,3-bisphosphoglycerate-dependent phosphoglycerate mutase | H-KYIENISDE-OH | 191-199 | **41** | LC |
| 15 | Probable phosphoketolase | H-IYLRSNPLMK-OH | 40-49 | **42** | BR, BD, BPG |
| 16 | Elongation factor Tu | H-GFDRDCPVV-OH | 164-172 | **43** | BPP (locus B) |
| 17 | Elongation factor Tu | H-DFVKNMITG-OH | 89-97 | **44** | BPP (locus L) |

### Example 2: In vitro activation of Oxytocin receptor (OT-R) by oxytocin-like bacterial peptide fragments

The present example studies the direct effect of peptides of SEQ ID NO:28 to SEQ ID NO: 44 on the Oxytocin receptor.

### Materials and Methods

The activation of OT-R by bacterial peptides was analyzed by the measurement of calcium release from HEK cells transfected with human OT-R

### Results

As a negative control, no activation of non-transfected HEK cells with OT or bacterial peptides was observed. In contrast, using OTR expressing HEK cells, OT and 10 out of 17 OT-like bacterial peptides have specifically activated OTR. Among these peptides there were: (i) 4 peptides (#1, 3, 7 and 8) from common proteins (PGK and EFTU), (ii) 3 peptides from Lactobacillus-specific proteins (#2, #6 and #7 as G6PI), 2 peptides from LP-specific proteins (#4 as GUAA and #5 as GLMS) and 1 peptide from LC-specific proteins (#14 as GPMA).

These results revealed that peptides derived from bacterial proteins specifically activate OTR even if their affinity is about 200-500 times lower than the OT peptide itself. Indeed, all these peptides have various micromolar EC₅₀ compared to the nanomolar EC₅₀ of OT. This characteristic and the maximal amplitude of Ca²⁺ release allow to highlight most efficient OT-like peptides ex. #1 (EFTU) and #3 (PGK). The only one common strain for both of these peptides, LS, was selected for in vivo studies. On the other hand, all synthetized peptides from EFTU (L locus) also specifically activated OTR. Because both target proteins EFTU and PGK were identified in LG, this strain was selected to be added to the mix with LS. Even if these two targets have been identified in BP as in LG, the EFTU sequence modifications in Bifidobacterium (L locus) reduced activity on OTR.

| **SEQ ID NO:** | **#Id** | **EC₅₀** | **Emax** |
|---|---|---|---|
| 28 | 1 | 2.77 x10⁻⁶± 0.79 | 273.8 ± 35.03 |
| 29 | 2 | 3.21 x10⁻⁶± 1.85 | 164.8 ± 107.9 |
| 30 | 3 | 8.62 x10⁻⁶± 0.50 | 302 ± 35.41 |
| 31 | 4 | 3.09 x10⁻⁶± 0.48 | 304.6 ± 29.70 |
| 32 | 5 | 2.70 x10⁻⁶± 1.03 | 272 ± 99.09 |
| 33 | 6 | 3.79 x10⁻⁶± 0.73 | 99.42 ± 6.77 |
| 34 | 7 | 2.57 x10⁻⁶± 0.13 | 262.8 ± 35.64 |
| 35 | 8 | 2.80 x10⁻⁶± 1.23 | 140 ± 71.48 |
| 36 | 9 | nd | 119.4 ± 47.19 |
| 37 | 10 | nd | 41.63 ±32.19 |
| 38 | 11 | nd | 4.65 ± 2.59 |
| 39 | 12 | nd | 5.25 ± 0.97 |
| 40 | 13 | nd | 17.92 ± 0.66 |
| 41 | 14 | 8.93 x10⁻⁶± 8.16 | 221.5 ± 10.40 |
| 42 | 15 | nd | 5.36 ± 0.31 |
| 43 | 16 | nd | 2.13 ± 0.83 |
| 44 | 17 | 7.52 x10⁻⁶± 0.10 | 135.3 ± 8.92 |
| | OT | 1.09 x10⁻⁹± 0.19 | 253.77 ± 5.25 |

Table 2 Table showing the ECso and Emax of each peptide as mean ± standard error (ND for not determined).

### Example 3: Effect of Mix 1 on anxiety and depression-like behavior: O-maze and water wheel tests

The present study aims at determining the effects of a Lactobacilli mixture, Mix 1, on the anxiety and depression-like behavior in mice during chronic mild stress.

### Materials and Methods

Mix 1 is a *Lactobacilli* mixture comprising *Lactobacillus Salivarius* and *Lactobacillus Gasseri* at 10¹⁰ CFU each. Mice were gavaged for 21 days. The number of mice was of n=8 per group.

The water wheel tests were performed in mice after 4 days of chronic mild stress.

### Results

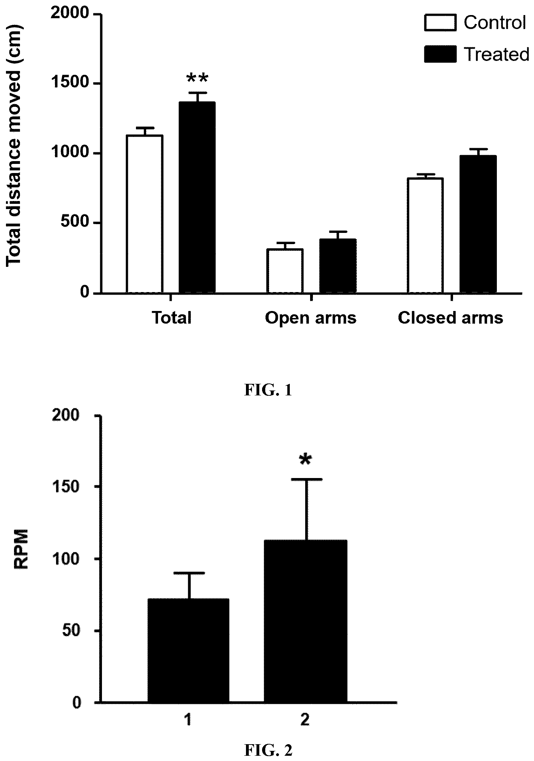

As presented on figure 1, increased exploratory activity was observed in the O-maze test for the group treated with Mix 1. Mix 1 promoted exploratory behavior related to total distance moved (cm) on the total platform in the treated group (black) as compared to control group (white).

As presented on figure 2, Mix 1 significantly increased the rotation activity (RPM = total Rotation Per Minute) in the treated group (2) as compared to the control group (1). Such increased rotation activity can be interpreted as reduced depressive-like behavior related to the motivation to escape from water.

Consequently, Mix 1 was demonstrated to improve exploratory behavior and water wheel rotation activity, thus assessing a reduced depressive-like behavior. Without willing to be bound by a theory, a composition comprising *Lactobacillus Salivarius* and *Lactobacillus Gasseri* exerts the above effects due to the presence of the most bioactive peptide sequences presented in table 2.

### Example 4: Effect of Mix 1 on depression-like behaviors: open-field test, tail suspension test (TST), forced swimming test (FST)

The effects of the Mix 1 of example 3 were also evaluated in additional tests to analyze depressive-like behavior

### Materials and Methods

. Mice were gavaged for 28 days and a protocol of chronic mild stress similar to that of the study of example 1 was applied. The basal stress was assessed by the open field test which measures rodents anxiety induced by open spaces. Total distance moved (cm) in open field was measured before and after 10 days of gavage in control group (white) and Lactobacilli treated group (black) (n=8 per group).

### Results

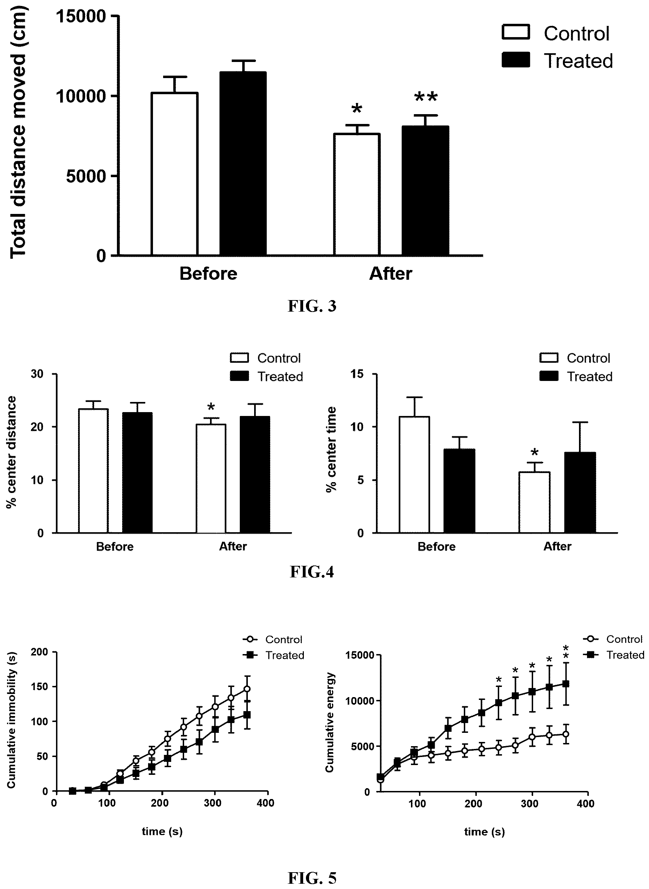

Gavage procedure in both groups significantly reduced locomotor activity, as is evidenced on Figure 3. However, in the control group, this reduction was directly linked to a decrease of the central zone as can be seen on Figure 4. In contrast, mice from the treated group spent the same time in the central area after treatment and moved on a similar distance. Taken together, these results suggest that the treatment with Mix 1 compensates the increased anxiety associated with open spaces during the basal level of stress.

To analyze the effect of treatment on anxiety and depression during chronic mild stress, additional behavioral tests were carried out.

The tail suspension test showed that mice from treated group were less immobile and deployed significantly more energy to escape as compared to the control group (Figure 5). Antidepressant effect is associated with decreased immobility and increased energy.

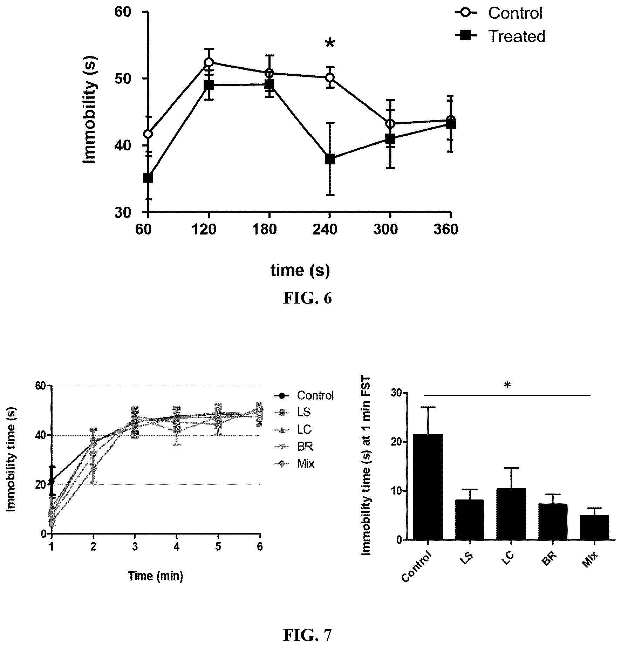

Likewise, the forced swimming test (FST) showed that the mice from treated group were overall more active and had a boost of energy after 4 minutes of swimming to escape from water (Figure 6). Antidepressant effect is associated with decreased immobility.

Thus, the results from these 2 tests showed that treatment with Mix1 reduced depressive-like behavior associated with despair in mice.

### Example 5: Anti-depressive effects of Mix 2

The present study aimed at assessing the anti-depressive effect of a second strain mixture (Mix 2) according to the invention, by comparison with the anti-depressive effect obtain with each individual strain of the mixture.

### Materials and Methods

Mix 2 is a mixture comprising *Lactobacillus salivarius, Lactobacillus camelliae* and *Bifidobacterium ruminantium* at a total of 10¹⁰ CFU, each strain representing a third of the concentration in Mix 2.

Mice were gavaged for 18 days in standard holding conditions and the effects of probiotic treatment on anxiety and depression were studied using the forced swimming test (FST).

### Results

As observed before, gavage reduced locomotor activity in all groups. In the FST, mice treated with Mix 2 were significantly more active than the non-treated controls (Figure 7). Mice receiving individual strains also displayed tendency of reduced immobility during the first minute of the test but it did not reach significance.

## Claims

1. A composition for use in the treatment of depression; said composition comprising *Lactobacillus salivarius* strain and at least one second *Lactobacillus* strain, and optionally at least one *Bifidobacterium* strain.

2. The composition for use according to claim **1,** wherein the at least one second *Lactobacillus* strain is selected from *Lactobacillus gasseri, Lactobacillus camelliae, Lactobacillus plantarum,* and/or *Lactobacillus reuteri.*

3. The composition for use according to claim **1** or claim **2,** wherein the at least one *Bifidobacterium* strain is selected from *Bifidobacterium ruminantium, Bifidobacterium dentium, Bifidobacterium pseudolongum* subsp. *pseudologum, Bifidobacterium pseudolongum* subsp. *globosum* and/or *Bifidobacterium adolescentis.*

4. The composition for use according to any one of claims **1** to **3,** wherein the composition comprises *Lactobacillus salivarius* and *Lactobacillus gasseri.*

5. The composition for use according to any one of claims **1** to **3,** wherein the composition comprises *Lactobacillus salivarius, Lactobacillus camelliae* and *Bifidobacterium ruminantium.*

6. Use of a composition for improving the mood and/or for relieving the every-day stress of a healthy subject, said composition comprising *Lactobacillus salivarius* strain and least one second *Lactobacillus* strain, and optionally at least one *Bifidobacterium* strain.

7. The use according to claim **6,** wherein the composition comprises *Lactobacillus salivarius* and *Lactobacillus gasseri,* or wherein the composition comprises *Lactobacillus salivarius, Lactobacillus camelliae* and *Bifidobacterium ruminantium.*

8. The use according to claim **6** or claim **7,** wherein the composition further comprises at least one nutraceutically acceptable excipient.

9. A composition comprising *Lactobacillus salivarius* strain, at least one second *Lactobacillus* strain and optionally at least one *Bifidobacterium* strain.

10. The composition according to claim **9,** wherein the at least one second *Lactobacillus* strain is *Lactobacillus gasseri.*

11. The composition according to claim **9** or claim **10,** wherein the at least one second *Lactobacillus* strain is *Lactobacillus camelliae* and wherein the composition comprises at least one *Bifidobacterium* strain selected from *Bifidobacterium ruminantium.*

12. The composition according to any one of claims **9** to **11,** said composition further comprising at least one plant or plant extract, preferably selected from the group consisting of *Rhodiola rosea, Withania somnifera, Melissa officinalis, Crocus sativus* and *Hypericum perforatum* and/or at least one magnesium supplement preferably selected from magnesium bound to an ammo acid or a magnesium salt selected from the group consisting of magnesium carbonate, magnesium oxide, magnesium acetate, magnesium ascorbate, magnesium citrate, magnesium gluconate, magnesium lactate, magnesium malate, magnesium pyrrolidone carboxylate, magnesium taurate, and magnesium threonate.

13. The composition according to any one of claims **9** to **12,** said composition being formulated in an oral dosage form selected from enterically-coated tablets and enterically-coated capsules, wherein the enteric-coating is a mixture comprising hydroxypropyl methyl cellulose and gellan gum.

14. The composition according to any one of claims **9** to **13,** said composition being formulated in a moisture-tight blister or container.

15. A composition comprising at least one bioactive peptide, wherein the at least one bioactive peptide is selected from the group consisting of peptides presenting from at least 80%, from at least 85%, from at least 90 from at least 95%, or 100% identity with any one the peptides of SEQ ID 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42, SEQ ID NO 43, and/or SEQ ID NO 44, for use in the prevention and/or treatment of depression.
